# EUROPÄISCHE PATENTANMELDUNG

(11) **EP 2 284 517 A1**
(43) Veröffentlichungstag der Anmeldung: **16.02.2011**
(21) Anmeldenummer: 10171080.4
(22) Anmeldetag: 28.07.2010
(51) Int. Cl.: G01N 21/05, G01N 21/27, G01N 21/31, G01N 21/85, G01N 33/14, G01N 21/15

(54) **Vorrichtung und Verfahren zur spektrometrischen Analyse eines Getränks**

(30) Priorität: 29.07.2009 DE 102009028067
(71) Anmelder: Dausch, Manfred, 76831 Göcklingen (DE)
(72) Erfinder: Dausch, Manfred, 76831 Göcklingen (DE)
(74) Vertreter: Kohler Schmid Möbus

(57) **Zusammenfassung**

Die Erfindung betrifft eine Vorrichtung zur spektrometrischen Analyse eines Getränks, umfassend: eine Messzelle (6), der das zu analysierende Getränk sowie das Getränk durchdringende elektromagnetische Strahlung zuführbar ist, sowie ein Spektrometer (7) zur Erfassung der durch das Getränk hindurch getretenen elektromagnetischen Strahlung. Die Messzelle (6) ist im Bereich einer Kreuzungsstelle (17) zwischen einer ersten Flüssigkeitsleitung (5) zum Durchfluss des zu analysierenden Getränks und einer zweiten Flüssigkeitsleitung (15) zum Durchfluss eines Reinigungs- und/oder Grundkalibrierungs- und/oder Nullpunktjustierungsfluids (14) angeordnet. Die Vorrichtung weist eine Ventileinrichtung (18a-d) für die wahlweise Zuführung des zu analysierenden Getränks oder des Reinigungs- und/oder Grundkalibrierungs- und/oder Nullpunktjustierungsfluids (14) zur Messzelle (6) auf. Die erste Flüssigkeitsleitung (5) weist einen Leitungsabschnitt (5a) für die Umgehung der Kreuzungsstelle (17) während der Zuführung des Reinigungs- und/oder Grundkalibrierungs- und/oder Nullpunktjustierungsfluids (14) zur Messzelle (6) auf. Die Erfindung betrifft auch ein zugehöriges Verfahren zur spektrometrischen Analyse eines Getränks.

## Beschreibung

### Vorrichtung und Verfahren zur spektrometrischen Analyse eines Getränks

Die vorliegende Erfindung betrifft eine Vorrichtung zur spektrometrischen Analyse eines Getränks. Die Erfindung betrifft auch ein zugehöriges Verfahren zur spektrometrischen Analyse eines Getränks.

Speziell in der Getränkeindustrie werden oft verschiedene Flüssigkeiten, wie zum Beispiel Wasser und mehrere Grundstoffe, in einem Mischbehälter oder einer Prozessleitung eines in-line-Mischers zusammengemischt und damit ein Fertiggetränk hergestellt, das dann mittels einer Abfüllmaschine in einzelne Behälter abgefüllt wird. Da beim Mischprozess verschiedene Fehlerquellen - wie zum Beispiel eine Fehlfunktion von Ventilen, mit denen die einzelnen Flüssigkeiten dem Mischbehälter oder der Prozessleitung (bei kontinuierlichen Mischprozessen) zugeführt werden, Druckschwankungen in den Zuführleitungen, fehlerhafte Messung der Menge der dem Mischbehälter oder der Prozessleitung zugeführten Flüssigkeiten, Fehler bei den lngredienzen, etc. - auftreten können, ist es zur Qualitätssicherung günstig, das Fertiggetränk nach Abschluss des Mischvorgangs spektrometrisch zu analysieren.

Aus der DE 10 2007 021 324 A1 der Anmelderin ist eine Vorrichtung zur Bestimmung des Mischverhältnisses einer aus zwei oder mehreren Einzelflüssigkeiten bestehenden Flüssigkeit bekannt, wobei die Vorrichtung eine Absorptionsmesszelle aufweist, der die zu analysierende Flüssigkeit zuführbar ist. Der Absorp-tionsmesszelle wird eine von einer Strahlungsquelle erzeugte, die Flüssigkeit durchdringende elektromagnetische Strahlung zugeführt. Das Absorptionsspektrum der zu analysierenden Flüssigkeit wird von einem Spektrometer erfasst und das Ausgangssignal des Spektrometers zu einer Auswerteinheit geführt, die aus dem aktuell gemessenen Absorptionsspektrum der Flüssigkeit und vorbestimmten Absorptionsspektren der Flüssigkeit und/oder der die Flüssigkeit ausbildenden Einzelkomponenten chemometrisch das Mischungsverhältnis dieser Einzelkomponenten bestimmt.

Zur Qualitätssicherung können auch andere Eigenschaften des Fertiggetränks, z.B. dessen Zuckergehalt (sog. Brix-Wert) oder dessen Säuregehalt (wie in der DE 10 2008 039 836.5 der Anmelderin dargestellt) auf spektrometrische Weise ermittelt werden. Hierzu wird in der Regel derart vorgegangen, dass der Charge des aktuell in einem Mischbehälter hergestellten Fertiggetränks eine Flüssigkeitsprobe über eine Probeentnahmeleitung mit Hilfe einer Pumpe entnommen und einer Messzelle zugeführt wird. Nach der Analyse einer bzw. mehrerer solcher Flüssigkeitsproben wird der Messzelle über eine weitere Zuführleitung ein Nullpunktsjustierungs-, Kalibrierungs- und/oder Reinigungsfluid, insbesondere Frischwasser zugeführt. Neben der Herstellung von Mischgetränken in einem diskontinuierlichen Prozess durch Mischen in einem Mischbehälter ist es in der Getränkeindustrie üblich, so genannte in-line bzw. Mehrkomponenten-Mischer zu verwenden, bei denen flüssige Komponenten, die aus einer oder mehreren Zuführleitungen stammen, kontinuierlich in einer Prozessleitung in einem konstanten Verhältnis gemischt werden, um sehr hohe Durchflussmengen zu erzielen.

Bei der oben beschriebenen diskontinuierlichen Probenentnahme mit sich anschließendem Reinigungs- und ggf. Kalibrierungs- bzw. Justierungsprozess ist es in der Regel jedoch schwierig, wenn nicht unmöglich, bei solchen kontinuierlich ablaufenden Mischprozessen ein fehlerhaft gemischtes Getränk im Prozess - wie z. B. durch Ventilschließung - zu stoppen und/oder durch Modifikation des Mischungsverhältnisses der Einzelflüssigkeiten oder auf andere Weise korrigierend einzugreifen. Wird zur Analyse des Getränks eine Messzelle direkt in die Rohrleitung integriert, tritt jedoch das Problem auf, dass die Messzelle während des kontinuierlich ablaufenden Mischprozesses ggf. einer Reinigung, einer Grundkalibrierung und/oder einer Nullpunktjustierung unterzogen werden muss.

Aus der EP 2 009 438 A1 sind ein Verfahren und eine Einrichtung zur Bestimmung des Wassergehalts in Mineralölen und ähnlichen Flüssigkeiten bekannt geworden. Dort wird eine Infrarotmesszelle verwendet, die kontinuierlich von dem zu untersuchenden Mineralöl durchströmt wird und deren Ausgangsmesswert bei einem gewählten Spektralwert ein Maß für den Wassergehalt des Mineralöls bildet. Die Infrarotmesszelle ist im Zentrum der Messanlage an einem Kreuzungspunkt zwischen einem Messkanal und einem Spülkanal angeordnet. Sowohl der Messkanal als auch der Spülkanal sind mit einer gemeinsamen Ablaufleitung verbunden, die in einem drucklosen Auffangbehälter endet.

Die US 6 458 213 B1 beschreibt ein Verfahren und eine Vorrichtung zur automatischen Reinigung von optoelektronischen Sensorsystemen zur Stoffanalyse in Flüssigkeiten und Gasen unter Ausnutzung von optischer Absorption und Fluoreszenz. Der Verschmutzungsgrad wird vom optoelektronischen Sensorsystem selbst detektiert und bei vorgewähltem Verschmutzungsgrad reinigt eine Reinigungsflüssigkeit die verschmutzungsempfindlichen optischen Komponenten. Die verbleibende Restverschmutzung wird erfasst und in der Signalauswertung quantitativ berücksichtigt und eliminiert. Einer Messzelle der Vorrichtung kann wahlweise über schaltbare Ventile ein Feuchtmittel, eine Reinigungsflüssigkeit oder Frischwasser zugeführt werden.

### Aufgabe der Erfindung

Es ist die Aufgabe der vorliegenden Erfindung, eine Vorrichtung und ein Verfahren der eingangs genannten Art derart weiterzubilden, dass eine fortlaufende spektrometrische Analyse eines (Misch-)getränks durchgeführt werden kann.

### Gegenstand der Erfindung

Diese Aufgabe wird erfindungsgemäß gelöst durch eine Vorrichtung, umfassend: eine Messzelle, der das zu analysierende Getränk sowie das Getränk durchdringende elektromagnetische Strahlung zuführbar ist, sowie ein Spektrometer zur Erfassung der durch das Getränk hindurch getretenen elektromagnetischen Strahlung, wobei die Messzelle an einer Kreuzungsstelle zwischen einer ersten Flüssigkeitsleitung zum Durchfluss des zu analysierenden Getränks und einer zweiten Flüssigkeitsleitung zum Durchfluss eines Reinigungs- und/oder Grundkalibrierungs- und/oder Nullpunktsjustierungsfluids angeordnet ist, wobei die Vorrichtung eine Ventileinrichtung für die wahlweise Zuführung des zu analysierenden Getränks oder des Reinigungs- und/oder Grundkalibrierungs- bzw. Nullpunktsjustierungsfluids zur Messzelle aufweist, und wobei die erste Flüssigkeitsleitung einen Leitungsabschnitt für die Umgehung der Kreuzungsstelle während der Zuführung des Reinigungs- und/oder Grundkalibrierungs- und/oder Nullpunktjustierungsfluids zur Messzelle aufweist.

Bei der erfindungsgemäßen Vorrichtung kann der Messzelle das zu analysierende (Misch-)Getränk kontinuierlich zugeführt werden, d.h. die Messzelle kann in-line in einer Rohrleitung eingesetzt werden, die von einem in-line-Mischer zu einer Abfüllmaschine führt. Durch das Anordnen der Messzelle an der Kreuzungsstelle kann für deren Reinigung und/oder Nullpunktsjustierung bzw. Grundkalibrierung in bestimmten Abständen ein Nullpunktjustierungs-, Grundkalibrierungs- und/oder Reinigungsfluid (eine Flüssigkeit und/oder ein Gas) zugeführt werden. Die Flüssigkeit zur Nullpunktjustierung (typischer Weise Frischwasser) kann hierbei in regelmäßigen Abständen, z.B. alle drei Stunden, kurz zugeführt werden, um den Nullpunkt für die spektrometrische Analyse eines bekannten Getränks neu einzustellen. Es versteht sich, dass zur Nullpunktsjustierung ggf. auch ein Haltezustand der gesamten Abfüllanlage genutzt werden kann, um die nach der Wiederinbetriebnahme der Anlage verfügbare Betriebszeit der Messzelle zu erhöhen. Bei der Flüssigkeit zur Grundkalibrierung kann es sich beispielsweise um ein neues Getränk handeln, das mit Hilfe der Messzelle spektrometrisch analysiert werden soll. Bei der Reinigungsflüssigkeit kann es sich um eine Spülflüssigkeit handeln, insbesondere um Wasser, das auch zur Nullpunktsjustierung verwendet werden kann. Als Reinigungsfluid kann z.B. bei hochempfindlichen Aseptikanlagen auch Sterilluft verwendet werden. Als Reinigungsfluide können ferner auch Heißdampf, Lauge, Säure, etc. verwendet werden. Derartige Reinigungsfiluide werden in der Regel zugeführt, wenn die gesamte Abfüllanlage gereinigt wird (sog. "Cleaning in Process" (CIP)-Prozess), d.h. wenn kein Getränk in der ersten Flüssigkeitsleitung vorhanden ist, das durch das Reinigungsfluid ggf. verunreinigt werden könnte. Während des CIP-Prozesses der Abfüllanlage kann auch durch die erste Flüssigkeitsleitung ein Reinigungsfluid, z.B. in Form von Heißdampf, einer Lauge oder einer Säure geleitet werden.

Das zwischen den Ventilen befindliche Volumen an der Kreuzungsstelle kann hierbei sehr klein gewählt werden, so dass der Wechsel zwischen der Zuführung von zu analysierendem Getränk und dem Reinigungs- und/oder Grundkalibrierungs-und/oder Nullpunktjustierungsfluid sehr schnell durchgeführt werden kann. Dadurch können die Totzeiten, in denen die Messzelle nicht für die Analyse des Getränks zur Verfügung steht, minimiert werden, so dass eine quasi-kontinuierliche spektrometrische Analyse des Getränks und damit eine fortlaufende Überwachung des Herstellungsprozesses sowie eine kontinuierliche Qualitätssicherung des zu analysierenden Getränks sichergestellt werden kann.

Der Leitungsabschnitt für die Umgehung der Kreuzungsstelle bildet einen Bypass für das zu analysierende Getränk, um sicherzustellen, dass auch während der Kalibrierung/Reinigung der Messzelle eine Abfüllung des Getränks in Behälter, z.B. in Flaschen erfolgen kann. Es versteht sich, dass der Bypass-Leitungsabschnitt auch geöffnet bleiben kann, während das zu analysierende Getränk der Messzelle zugeführt wird. Durch das Vorsehen des die Messzelle umgehenden Leitungsabschnitts kann der Rohrquerschnitt des Leitungsabschnitts mit der Messzelle verhältnismäßig klein dimensioniert werden, so dass sich das an der Kreuzungsstelle vorhandene Totvolumen weiter reduziert.

Bei einer vorteilhaften Ausführungsform weist die Ventileinrichtung zwei Ventilpaare zum wahlweisen Verbinden oder Trennen der Kreuzungsstelle mit / von der ersten oder der zweiten Flüssigkeitsleitung auf. Die Ventilpaare müssen hierbei so ausgebildet sein, dass sie ein sicheres fluiddichtes Abtrennen der Kreuzungsstelle von der jeweiligen Flüssigkeitsleitung ermöglichen. Die Ventile können zu diesem Zweck z.B. als aseptische Ventile mit Schraubenfedern zur Ventilschließung ausgelegt sein. Falls auch während des Abfüllprozesses der Messzelle ein Reinigungsfluid z.B. in Form einer Lauge zugeführt werden soll, können ggf. Doppelsitz-Ventile verwendet werden, um sicherzustellen, dass das Reinigungsfluid nicht in das zu analysierende Getränk gelangen und dieses verunreinigen kann.

Bei einer weiteren Ausführungsform umfasst die Vorrichtung weiterhin einen Behälter zur Aufnahme des Reinigungs- und/oder Grundkalibrierungs- und/oder Nullpunktjustierungsfluids, der mit der zweiten Flüssigkeitsleitung verbindbar bzw. verbunden und insbesondere oberhalb der Kreuzungssfielle angeordnet ist. Der Behälter dient der Speicherung des jeweils zum Spülen oder zur Kalibrierung der Messzelle benötigten Fluids. Der Behälter kann hierbei insbesondere oberhalb der Kreuzungsstelle angeordnet sein, so dass der Druck der über der Messzelle angeordneten Fluidsäule für die Spülung der Messzelle mit dem Reinigungs- und/oder Grundkalibrierungs- und/oder Nullpunktjustierungsfluid ausreichend ist und ggf. auf eine Pumpe für den Spülvorgang verzichtet werden kann. Die Zuführung des Grundkalibrierungs- und/oder Nuflpunkfijustierungsfluids zur Kreuzungsstelle kann hierbei bevorzugt von unten erfolgen, sodass das ggf. in der Kreuzungsstelle vorhandenes Gas (typischer Weise Umgebungsluft) nach oben verdrängt werden kann. Die oben aus der Kreuzungsstelle austretende Flüssigkeit wird über weitere Rohrleitungen einem Abfluss zugeführt. Auch unter Druck stehende Reinigungsflüssigkeit (z.B. Wasser oder ein CIP-Medium) kann der Kreuzungsstelle über ein Druckventil von unten zugeführt werden.

In einer vorteilhaften Ausführungsform ist die Messzelle als Absorptionsmesszelle ausgebildet, die bevorzugt einen ersten Kollimator, dem elektromagnetische Strahlung zuführbar ist, und einen zweiten Kollimator aufweist, mit dem durch das Getränk bzw. das Grundkalibrierungs- und/oder Nullpunktjustierungsfluid hindurchgetretene elektromagnetische Strahlung erfassbar ist. Die Kollimatoren weisen hierbei optische Fenster auf, welche die Wellenlängen der verwendeten elektromagnetischen Strahlung nur geringfügig dämpfen und resistent gegen die verwendeten Reinigungsmittel sind. Die Absorptionsmesszelle kann hierbei insbesondere stabförmig ausgebildet sein, wobei die beiden Kollimatoren z.B. einander gegenüberliegend im Bereich eines Stab-Endes angeordnet sein können. Insbesondere können hierbei eine Strahlungsquelle zur Erzeugung der elektromagnetischen Strahlung mit der Absorptionsmesszelle und/oder die Absorptionsmesszelle mit dem Spektrometer über einen Lichtwellenleiter verbunden sein. Es versteht sich, dass auch andere Typen von Messzellen, z.B. eine sog. ATR("attenuated total reflection")-Sonde verwendet werden kann. Letztere bietet den Vorteil, dass sie auch bei hochviskosen Flüssigkeiten, Flüssigkeiten mit einem hohen Absorptionskoeffizenten und bei Flüssigkeiten, die einen hohen Streueffekt vorweisen, sehr gute Resultate liefert.

Bei einer Weiterbildung der Messzelle verläuft die Durchflussrichtung des Getränks zwischen den Kollimatoren unter einem Winkel, insbesondere unter einem Winkel von 45°, zur ersten Flüssigkeitsleitung und zur zweiten Flüssigkeitsleitung. Die beiden Flüssigkeitsleitungen verlaufen typischer Weise zumindest im Bereich der Kreuzungsstelle in einer gemeinsamen (in der Regel vertikalen) Ebene. Durch die Ausrichtung des Durchtrittsspalts zwischen den Kollimatoren unter einem Winkel zu den beiden Flüssigkeitsleitungen, der insbesondere um ca. 45° liegen kann, wird sichergestellt, dass sowohl das durch die erste Flüssigkeitsleitung fließende Getränk als auch das durch die zweite Flüssigkeitsleitung fließende Reinigungs- und/oder Grundkalibrierungs- und/oder Nullpunktjustierungsfluid schnell durch die Messzelle hindurch strömen können. Es versteht sich, dass auch bei Messzellen von anderem Typ eine Ausrichtung des durchströmten Kanals bzw. Spalts unter einem Winkel zu den beiden Flüssigkeitsleitungen günstig sein kann.

In einer weiteren Ausführungsform weist die Vorrichtung eine Strahlungsquelle zur Erzeugung der elektromagnetischen Strahlung auf, die bevorzugt in einem Wellenlängenbereich zwischen 200 nm und 1000 nm liegt. Es versteht sich, dass die Strahlungsquelle nicht zur Erzeugung von Strahlung im gesamten angegebenen Wellenlängenbereich ausgelegt sein muss. Insbesondre kann die Strahlungsquelle zur Erzeugung von Strahlung in einem Wellenlängenbereich zwischen 200 nm und 700 nm ausgelegt sein, in dem viele bei der Spektroskopie von Flüssigkeiten auftretenden Absorptionsbanden liegen. Der vom Spektrometer erfassbare Wellenlängenbereich ist hierbei typischer Weise auf den Spektralbereich, in dem die gewünschten Absorptionsbanden der zu messenden Flüssigkeit liegen, abgestimmt. Das Spektrometer kann hierbei insbesondere breitbandig ausgelegt sein oder es können verschiedene Spektrometermodule (UV/VIS, VIS, VIS/NIR, NIR) verwendet werden, um ein Spektrum der UVIVIS/NIR-Strahlung im gesamten oben genannten Wellenlängenbereich und ggf. auch darüber hinaus (z.B. bis ca. 2500 nm) zu erfassen.

Ein weiterer Aspekt der Erfindung betrifft ein Verfahren zur spektrometrischen Analyse eines Getränks, umfassend: Zuführen des zu analysierenden Getränks sowie von das Getränk durchdringender elektromagnetischer Strahlung zu einer Messzelle, die im Bereich einer Kreuzungsstelle zwischen einer ersten Flüssigkeitsleitung zum Durchfluss des zu analysierenden Getränks und einer zweiten Flüssigkeitsleitung zum Durchfluss eines Reinigungs- und/oder Grundkalibrierungs-und/oder Nullpunktjustierungsfiluids angeordnet ist, sowie Erfassen der durch das zu analysierende Getränk hindurch getretenen elektromagnetischen Strahlung in einem Spektrometer, wobei die Verbindung der Messzelle mit der ersten Flüssigkeitsleitung unterbrochen wird, wenn der Messzelle das Reinigungs- und/oder Grundkalibrierungs- und/oder Nullpunktjustierungsfluid über die zweite Flüssigkeitsleitung zugeführt wird, und wobei während des Zuführens des Reinigungs- und/oder Grundkalibrierungs- und/oder Nulfpunktjusfiierungsfluids zur Messzelle das zu analysierende Getränk durch einen die Kreuzungsstelle umgehenden Leitungsabschnitt der ersten Flüssigkeitsleitung geleitet wird, um einen kontinuierlichen Durchfluss des zu analysierenden Getränks sicherzustellen. Es versteht sich, dass ein Durchfluss des zu analysierenden Getränks durch den die Kreuzungsstelle umgehenden Leitungsabschnitt auch erfolgen kann, während die Messzelle mit der ersten Flüssigkeitsleitung in Verbindung steht.

Bei dem erfindungsgemäßen Verfahren wird die Verbindung der Messzelle zur ersten Flüssigkeitsleitung unterbrochen, wenn die Messzelle mit einem Fluid, insbesondere mit einem Reinigungs-, Grundkalibrierungs- und/oder Nullpunktjustierungsfluid beaufschlagt wird. Durch die Anordnung der Messzelle am Kreuzungspunkt zwischen den beiden Flüssigkeits- bzw. Rohrleitungen kann die Zu- bzw. Abführung des Fluids zur Messzelle besonders schnell erfolgen, so dass die Messzelle die Analyse des Getränks schnell wieder aufnehmen kann. Das wahlweise Verbinden oder Trennen der Kreuzungsstelle bzw. der Messzelle von der ersten oder der zweiten Flüssigkeitsleitung kann hierbei insbesondere durch Ansteuern (Öffnen / Schließen) von jeweils zwei Ventilpaaren z.B. mittels eines ASI-Busses erfolgen.

Bei einer Variante wird die Messzelle mindestens 98 %, bevorzugt mindestens 99,9 % ihrer Betriebszeit von zu analysierendem Getränk durchströmt. Die Messzelle wird nur während kurzer, typischer Weise regelmäßiger Zeitintervalle mit Nullpunktjustierungsflüssigkeit gespült, so dass eine quasi-kontinuierliche Erfassung des Spektrums des zu analysierenden Getränks erfolgen kann. Die Verfügbarkeit kann auch nahe an der 100 %-Marke liegen, wenn die Nullpunktjustierung während eines Anlagen-Stopps durchgeführt wird. Hierdurch kann eine kontinuierliche Qualitätskontrolle des zu analysierenden Getränks, z.B. eines abzufüllenden Mischgetränks, sichergestellt werden.

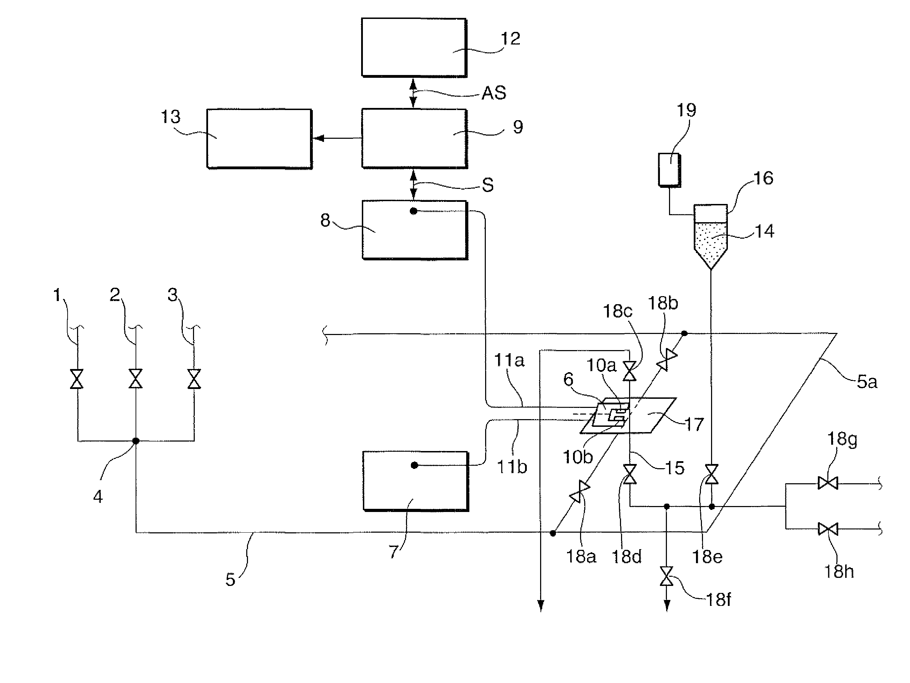

Weitere Vorteile der Erfindung ergeben sich aus der Beschreibung und der Zeichnung. Ebenso können die vorstehend genannten und die noch weiter aufgeführten Merkmale je für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

Es zeigt:
Figur eine schematische Darstellung einer Ausführungsform einer erfindungsgemäßen Vorrichtung zur spektroskopischen Analyse eines Getränks.

Die Figur zeigt eine Vorrichtung zur spektroskopischen Analyse eines aus mehreren Einzelflüssigkeiten bestehenden Mischgetränks. Die Vorrichtung weist einen in-line Mischer **4** auf, dem über drei Zuführleitungen **1, 2, 3** die zur Herstellung des Mischgetränks - z. B. eines Fertiggetränks - dienenden Einzelflüssigkeiten zugeführt werden. Dem in-line Mischer 4 werden die Einzelflüssigkeiten über Ventile zugeführt, welche die Einstellung eines konstanten Mischungsverhältnisses erlauben. Das Mischgetränk wird von dem in-line Mischer 4 über eine Zuführungsleitung **5** einer nicht näher dargestellten Abfüllamaschine zugeführt. Es versteht sich, dass auch mehr oder weniger als die beispielhaft gezeigten drei Zuführleitungen 1, 2, 3 zur Herstellung des Mischgetränks verwendet werden können.

Um nun für das in dem in-line Mischer 4 erzeugte Mischgetränk eine spektrometrische Analyse durchzuführen, z.B. um das Mischungsverhältnis der über die Zuführleitungen 1, 2, 3 zugeführten Einzelflüssigkeiten in dem Mischgetränk oder um den Säuregehalt in dem Mischgetränk zu bestimmen, ist in der Abfüllmaschinen-Zuführleitung 5 eine Messzelle **6** angeordnet.

Der Messzelle 6 wird von einer Strahlungsquelle **7** erzeugte Strahlung zugeführt, welche das in der Messzelle 6 befindliche Getränk beaufschlagt. Die Strahlungsquelle 7 ist im vorliegenden Beispiel als eine UV/VIS/NIR-Strahlungsquelle ausgebildet, welche eine elektromagnetische Strahlung erzeugt, deren Wellenlänge im ultravioletten, sichtbaren und im NIR-Bereich, also typischerweise im Bereich von ca. 200 nm - 1.000 nm, ggf. auch darüber liegt. Eine derartige UV/VIS/NIR-Strahlungsquelle ist bekannt und wird daher nicht im Einzelnen beschrieben. Die von dem Kollimator 10a aufgenommene - durch das Getränk geschwächte - Strahlung wird zu einem Spektrometer **8** geführt, durch welches das Spektrum des zu analysierenden Getränks erzeugt und erfasst wird.

Die Messzelle 6 ist bei dem hier beschriebenen Ausführungsbeispiel als Absorptionsmesszelle ausgebildet und weist zwei einander gegenüberliegende Kollimatoren **10a, 10b** auf, deren optische Fenster vorzugsweise derart ausgebildet sind, dass sie die bei der nachfolgend beschriebenen Absorptionsmessung benötigten, im UV/VIS/NIR-Bereich liegenden optischen Wellenlängen wenig dämpfen.

Einem der Kollimatoren 10b wird über einen Lichtwellenleiter **11b** die von der Strahlungsquelle 7 erzeugte elektromagnetische Strahlung zugeführt. Nachdem die Strahlung das zwischen den beiden Kollimatoren 10a, 10b befindliche Getränk durchquert hat, wird sie in einem weiteren Kollimator 10a empfangen und über einen weiteren Lichtwellenleiter **11a** zum Spektrometer 8 geführt und darin das Absorptionsspektrum gemessen. Das Ausgangssignal **S** des Spektrometers 8 wird einer Auswerteeinheit **9** zugeführt, deren Ausgangssignal **AS** einer Prozesssteuereinrichtung **12** zugeführt und vorzugsweise auf einem Bildschirm **13** wiedergegeben wird.

In der Auswerteinheit 9 kann beispielsweise das Mischungsverhältnis der Einzelflüssigkeiten bestimmt werden, wie in der DE 10 2007 021 324 der Anmelderin näher ausgeführt ist, die bezüglich dieses Aspekts durch Bezugnahme zum Inhalt dieser Anmeldung gemacht wird. Die Prozesssteuereinrichtung 12 kann z.B. auch der Steuerung/Regelung der Ventile für die Zuführleitungen 1, 2, 3, zum in-line Mischer 4 in Abhängigkeit vom ermittelten Mischungsverhältnis dienen.

Im in-line Mischer **4** wird ein kontinuierlicher Mischprozess durchgeführt, d.h. die Zuführungsleitung 5 wird kontinuierlich mit dem zu analysierenden Getränk beschickt. Das Spektrometer 8, das mit der in der Zuführungsleitung 5 angeordneten Messzelle 6 verbunden ist, ist so ausgelegt, dass in kurzen Abständen (z.B. im Millisekundenbereich) jeweils ein vollständiges Spektrum des Getränks erfasst werden kann.

Beim Betrieb der Messzelle 6 tritt nun das Problem auf, dass sich in dem Getränk vorhandene Schwebeteile an den optischen Fenstern der Kollimatoren 10a, 10b ablagern und die Messung ggf. beeinträchtigen können. Daher ist es günstig, der Messzelle 6 ein Reinigungfluid, z.B. in Form von Frischwasser zugeführt wird. Auch sollte von Zeit zu Zeit eine Nullpunktjustierung mit einer Nullpunktsjustierungsflüssigkeit, z.B. mit Frischwasser, durchgeführt werden.

Um diese und ggf. weitere Reinigungs- bzw. Grundkalibrierungs- und/oder Nullpunktjustierungsfluide **14** der Messzelle 6 zuzuführen, weist die Vorrichtung eine Zuführleitung **15** sowie einen Behälter **16** auf, der zur Aufnahme einer derartigen Grundkalibrierungs- und/oder Nullpunktjustierungs bzw. Reinigungsflüssigkeit und/oder weiterer Flüssigkeiten dient. Die Messzelle 6 ist hierbei im Bereich einer Kreuzungsstelle **17** (Rohrkreuzung) zwischen der Zuführungsleitung 5 als erster Flüssigkeitsleitung und der Zuführleitung 15 als zweiter Flüssigkeitsleitung angeordnet. Eine Ventileinrichtung mit vier Ventilen **18a-d** dient der wahlweisen Zuführung des zu analysierenden Getränks oder des Reinigungs- und/oder Grundkalibrierungs- und/oder Nullpunktjustierungsfluids 14 zur Messzelle 6.

Hierbei dient ein erstes, an der Zuführungsleitung 5 angeordnetes Ventilpaar 18a, 18b dem wahlweisen Verbinden oder Trennen der Kreuzungsstelle 17 mit bzw. von der Zuführungsleitung 5. Ein zweites Ventilpaar 18c, 18d dient dem wahlweisen Verbinden mit bzw. dem Trennen der Kreuzungsstelle 17 von der Zuführleitung 15. Jedes der Ventile 18a-d ist hierbei von der Prozesssteuereinrichtung 12 individuell ansteuerbar (z.B. über einen ASI-Bus). Das an der Kreuzungsstelle 17 zwischen den Ventilen 18a-d gebildete Volumen, in dem sich die Messzelle 6 befindet, ist in der Figur schematisch durch ein Rechteck angedeutet (das in der Realität deutlich kleiner ist). Um zu gewährleisten, dass sowohl das die erste Flüssigkeitsleitung 5 durchströmende Getränk als auch ein die zweite Flüssigkeitsleitung 15 durchströmendes Fluid durch den zwischen den Kollimatoren 10a, 10b gebildeten Spalt bzw. Strömungskanal schnell hindurch treten kann, ist der Spalt und damit die Durchflussrichtung unter einem Winkel von ca. 45° zu den beiden Flüssigkeitsleitungen 5, 15 angeordnet. Eine solche, im Wesentlichen symmetrische Ausrichtung der Messzelle 6 zu den Flüssigkeitsleitungen 5, 15 ist auch deshalb günstig, weil die Strömungsrichtung beim Durchströmen der Messzelle für das Grundkalibrierungsund/oder Nullpunktjustierungsfluid sich von denen für das Getränk nur geringfügig unterscheidet.

Die Ansteuerung der Ventile 18a-d für das Durchführen eines Spülprozesses kann z.B. wie folgt erfolgen: Zunächst werden die Ventile 18a, 18b in der Zuflussleitung 5 geschlossen. Dann wird das noch im Bereich der Kreuzungsstelle 17 vorhandene Getränk durch Öffnen des an der Zuführleitung 15 unterhalb der Kreuzungsstelle 17 vorgesehenen Ventils 18d sowie eines weiteren Ventils 18f einem in der Figur durch einen Pfeil angedeuteten Abfluss zugeführt. Nachfolgend wird das oberhalb der Kreuzungsstelle 17 angeordnete Ventil 18c sowie ein weiteres Ventil 18e geöffnet, um aus dem Behälter 16 die Reinigungsflüssigkeit 14, im vorliegenden Fall Frischwasser, der Kreuzungsstelle 17 bzw. der Messzelle 6 zu- und über das obere Ventil 18c einem durch einen Pfeil angedeuteten Abfluss zuzuführen. Die Zuführleitung 15 dient hierbei als Fallrohr, die ein schnelles Spülen der Messzelle 17 mittels der im Behälter 16 vorgesehenen Flüssigkeit 14 ermöglicht, ohne dass hierzu eine Pumpe erforderlich ist. Nach dem Reinigen wird zunächst das obere Ventil 18c und nach dem Ablassen der Reinigungsflüssigkeit auch das untere Ventil 18d geschlossen, bevor die in der Zuführungsleitung 5 angeordneten Ventile 18a, 18b geöffnet werden, um den Durchfluss des zu analysierenden Getränks und deren weitere Analyse zu ermöglichen.

Um zu gewährleisten, dass das zu analysierende Getränk auch während des Reinigungsprozesses durch die Zuführungsleitung 5 zur Abfüllanlage gelangen kann, ist ein die Kreuzungsstelle 17 umgehender Leitungsabschnitt **5a** (By-Pass) an der Zuführungsleitung 5 vorgesehen. Im vorliegenden Beispiel strömt das zu analysierende Getränk dauerhaft durch den die Kreuzungsstelle 17 umgehenden Leitungsabschnitt 5a, der einen größeren Rohrquerschnitt aufweist als der Rohrquerschnitt des parallelen Leitungsstücks mit der Kreuzungsstelle 17, um das Volumen zwischen den Ventilen 18a-d möglichst klein zu halten. Hierbei können die durch die Rohrquerschnitte festgelegten Strömungswiderstände des Abschnitts mit der Kreuzungsstelle 17 und des diese umgehenden Abschnitts 5a relativ zueinander so eingestellt werden, dass z.B. mehr als 80 %, ggf. auch mehr als 90 % des zu analysierenden Getränks durch den umgehenden Leitungsabschnitt 5a fließen.

Es versteht sich, dass ggf. eine oder mehrere Pumpen in der Vorrichtung vorgesehen sein können, um über weitere Zuführungsleitungen Reinigungs- und/oder ein Grundkalibrierungs- und/oder Nullpunktjustierungsfluid zu dem Behälter 16 zuzuführen. Auch können weitere, mit der Zuführleitung 5 verbindbare Behälter in der Vorrichtung vorgesehen sein. Der Behälter 16 kann ferner einen Füllstandsmesser aufweisen, der mit der Prozesssteuerung 12 verbunden werden kann, um den Füllstand des Behälters 16 auf dem Bildschirm 13 anzuzeigen. Die beim Entleeren der Flüssigkeit 14 in den Behälter 16 nachströmende Luft wird diesem im vorliegenden Beispiel über einen Sterilfilter **19** zugeführt, um Verunreinigungen der Reinigungs- bzw. Kafibrierungsfilüssigkeit durch in der Umgebungsluft vorhandene Verunreinigungen zu verhindern.

Es versteht sich, dass der Kreuzungsstelle 17 Fluide auch auf andere Weise als über den Behälter 16 zugeführt werden können, insbesondere wenn diese Fluide druckbeaufschlagt zugeführt werden sollen, was insbesondere bei der Zuführung von Reinigungsfiiuiden der Fall sein kann. Wie in der Figur gezeigt, kann beispielsweise über ein erstes Druckventil 18g Wasser und über ein zweites Druckventil 18h ein CIP-Medium, z.B. einer Säure oder einer Lauge, über das untere Ventil 18d der Kreuzungsstelle 17 zugeführt werden. Die Druckbeaufschlagung der Reinigungsfluide kann hierbei über (nicht gezeigte) Pumpen erfolgen.

Wie bereits weiter oben ausgeführt sollte das Leitungsvolumen an der zwischen den Ventilen 18a-d liegenden Kreuzungsstelle 17 möglichst klein gewählt werden, um einen schnellen Wechsel zwischen Reinigungs- bzw. Grundkalibrierungs- und/oder Nullpunktsjustierungsflüssigkeit 14 und zu analysierende Getränk zu gewährleisten. Zu diesem Zweck sollte die Messzelle 6 eine kompakte Bauart aufweisen, was insbesondere sichergestellt werden kann, wenn die Messzelle 6 z.B. stabförmig ausgebildet und über eine Rohröffnung in den Bereich der Kreuzungsstelle 17 eingebracht werden kann. Bei kleinem zu spülenden Volumen im Bereich zwischen den Ventilen 18a-d kann die Messzelle 6 mehr als 99% ihrer Betriebszeit zur Analyse des Getränks verwendet werden. Somit kann auf die hier beschriebene Weise eine automatisierte, quasi-kontinuierliche Analyse des zu analysierenden Getränks - insbesondere eines Mischgetränks - und damit eine kontinuierlich Qualitätssicherung erreicht werden.

## Patentansprüche

1. Vorrichtung zur spektrometrischen Analyse eines Getränks, umfassend:
eine Messzelle (6), der das zu analysierende Getränk sowie das Getränk durchdringende elektromagnetische Strahlung zuführbar ist, sowie ein Spektrometer (7) zur Erfassung der durch das Getränk hindurch getretenen elektromagnetischen Strahlung, wobei die Messzelle (6) im Bereich einer Kreuzungsstelle (17) zwischen einer ersten Flüssigkeitsleitung (5) zum Durchfluss des zu analysierenden Getränks und einer zweiten Flüssigkeitsleitung (15) zum Durchfluss eines Reinigungs- und/oder Grundkalibrierungs- und/oder Nullpunktjustierungsfluids (14) angeordnet ist, wobei die Vorrichtung eine Ventileinrichtung (18) für die wahlweise Zuführung des zu analysierenden Getränks oder des Reinigungs- und/oder Grundkalibrierungs- und/oder Nullpunktjustierungsfluids (14) zur Messzelle (6) aufweist, und wobei die erste Flüssigkeitsleitung (5) einen Leitungsabschnitt (5a) für die Umgehung der Kreuzungsstelle (17) während der Zuführung des Reinigungs- und/oder Grundkalibrierungs- und/oder Nullpunktjustierungsfluids (14) zur Messzelle (6) aufweist.

2. Vorrichtung nach Anspruch 1, bei der die erste Flüssigkeitsleitung (5) eine Zuführungsleitung von einem in-line Mischer (4) zu einer Abfüllmaschine ist.

3. Vorrichtung nach einem der vorhergehenden Ansprüche, bei der die Ventileinrichtung (18) zwei Ventilpaare (18a, 18b; 18c, 18d) zum wahlweisen Verbinden oder Trennen der Keuzungsstelle (17) von der ersten oder der zweiten Flüssigkeitsleitung (5, 15) aufweist.

4. Vorrichtung nach einem der vorhergehenden Ansprüche, weiter umfassend einen Behälter (6) zur Aufnahme des Grundkalibrierungs- und/oder Nullpunktjustierungs- und/oder Reinigungsfluids (14), der mit der zweiten Flüssigkeitsleitung (15) verbunden und insbesondere oberhalb der Kreuzungsstelle (17) angeordnet ist.

5. Vorrichtung nach einem der vorhergehenden Ansprüche, bei der die Messzelle (6) als Absorptionsmesszelle ausgebildet ist, die bevorzugt einen ersten Kollimator (10b), dem elektromagnetische Strahlung zuführbar ist, und einen zweiten Kollimator (10a), mit dem durch das Getränk hindurchgetretene elektromagnetische Strahlung erfassbar ist, aufweist.

6. Vorrichtung nach Anspruch 5, bei der die Durchflussrichtung des Getränks zwischen den Kollimatoren (10a, 10b) unter einem Winkel, insbesondere unter einem Winkel von 45°, zur ersten Flüssigkeitsleitung (5) und zur zweiten Flüssigkeitsleitung (15) verläuft.

7. Vorrichtung nach einem der vorhergehenden Ansprüche, weiter umfassend eine Strahlungsquelle (7) zur Erzeugung der elektromagnetischen Strahlung, bevorzugt in einem Wellenlängenbereich zwischen 200 nm und 1000 nm.

8. Verfahren zur spektrometrischen Analyse eines Getränks, umfassend:
Zuführen des zu analysierenden Getränks sowie von das Getränk durchdringender elektromagnetischer Strahlung zu einer Messzelle (6), die im Bereich einer Kreuzungsstelle (17) zwischen einer ersten Flüssigkeitsleitung (5) zum Durchfluss des zu analysierenden Getränks und einer zweiten Flüssigkeitsleitung (15) zum Durchfluss eines Reinigungs- und/oder Grundkalibrierungs- und/oder Nullpunktjustierungsfluids (14) angeordnet ist, sowie Erfassen der durch das zu analysierende Getränk hindurch getretenen elektromagnetischen Strahlung in einem Spektrometer (7), wobei die Verbindung der Messzelle (6) zur ersten Flüssigkeitsleitung (5) unterbrochen wird, wenn der Messzelle (6) über die zweite Flüssigkeitsleitung (15) das Reinigungs- und/oder Grundkalibrierungs- und/oder Nullpunktjustierungsfluid (14) zugeführt wird, und
wobei während des Zuführens des Grundkalibrierungs- und/oder Nullpunktjustierungs- und/oder Reinigungsfluids zur Messzelle (6) das zu analysierende Getränk durch einen die Kreuzungsstelle (17) umgehenden Leitungsabschnitt (5a) der ersten Flüssigkeitsleitung (5) geleitet wird.

9. Verfahren nach Anspruch 8, bei dem die Messzelle (6) mindestens 98%, bevorzugt mindestens 99,9 % ihrer Betriebszeit von zu analysierendem Getränk durchströmt wird.
